# EUROPÄISCHE PATENTSCHRIFT

(11) **EP 1 586 343 B1**
(45) Veröffentlichungstag und Bekanntmachung des Hinweises auf die Patenterteilung: **26.09.2007**
(21) Anmeldenummer: 05001304.4
(22) Anmeldetag: 22.01.2005
(51) Int. Cl.: A61M 16/00

(54) **Vorrichtung zur Beatmung sowie Verfahren zur Steuerung eines Beatmungsgerätes**
Breathing device and method of controlling a breathing device
Appareil respiratoire et méthode de commande d'un appareil respiratoire

(30) Priorität: 14.04.2004 DE 102004018029
(43) Veröffentlichungstag der Anmeldung: 19.10.2005
(73) Patentinhaber: Weinmann Geräte für Medizin GmbH & Co. KG, 22767 Hamburg (DE)
(72) Erfinder: Göbel, Christoph, 22529 Hamburg (DE); Tiemann, Björn, 22926 Ahrensburg (DE); Wedler, Wolfgang, 21147 Hamburg (DE)
(74) Vertreter: Klickow, Hans-Henning

(56) Entgegenhaltungen:
- EP-A- 0 943 353
- EP-A- 1 346 743
- EP-A- 1 371 384
- US-A1- 2002 053 345

## Beschreibung

Die Erfindung betrifft eine Vorrichtung zur Beatmung nach dem Oberbegriff des Anspruchs 1.

Eine derartige Vorrichtung kann beispielsweise im Zusammenhang mit einer sogenannten Bilevel-Beatmung eingesetzt werden. Das Beatmungsgerät stellt hierbei einen Inspirationsdruck sowie einen Exspirationsdruck bereit. Grundsätzlich wird zwischen einer kontrollierten Beatmung, einer assistierten Beatmung sowie Beatmungsmischformen unterschieden.

Bei der kontrollierten Beatmung werden die Beatmungsparameter während der Inspiration vollständig vom Beatmungsgerät bestimmt. Es liegen grundsätzlich zwei Formen der kontrollierten Beatmung vor, nämlich volumenkontrollierte und druckkontrollierte Beatmung. Bei der volumenkontrollierten Beatmung wird Atemzug für Atemzug ein definiertes Tidalvolumen verabreicht, der Druck kann zwischen den Atemhüben widerstandsabhängig variieren. Grundlage für die Umschaltung in die Exspirationsphase ist die Erreichung eines vorgegebenen Zielvolumens oder einer Inspirationszeit. Bei der druckkontrollierten Beatmung wird der Therapiedruck konstant gehalten. Das resultierende Volumen kann abhängig von atemmechanischen Parametern variieren. Die Umschaltung in die Exspirationsphase erfolgt zeitgesteuert.

Bei einer volumenkontrollierten Beatmung steht die hinreichende Applizierung eines definierten Gasvolumens pro Atemhub im Vordergrund. Die druckkontrollierte Beatmung besitzt den Vorteil, daß bei geeigneter Parametereinstellung keine unzulässigen Druckspitzen das Lungengewebe schädigen. Allerdings ist das applizierte Tidalvolumen stark abhängig von der Mitarbeit des Patienten bei der Atmung und von den atemmechanischen Größen Resistance und Lungen- Compliance. Andererseits ist die volumenkontrollierte Beatmung grundsätzlich primär bei der invasiven Beatmung sinnvoll, da bei der Maskenbeatmung Leckagen auftreten, die den eigentlichen Regelparameter auch bei einer Kalkulation von auftretenden Leckagen ungenau machen.

Bei einer druckregulierten und volumenkontrollierten Beatmung werden Vorteile der volumenkontrollierten Beatmung mit den Vorteilen der druckkontrollierten Beatmung gekoppelt. Das applizierte Beatmungsvolumen ist abhängig von mechanischen Eigenschaften der Lunge und vom Beatmungsdruck. Fällt das Volumen unter einen voreingestellten wert, wird in den folgenden Atemhüben der inspiratorische Druck in kleinen Schritten angehoben, bis das Zielvolumen erreicht ist.

Bei der assistierten Beatmung kann der Patient den Zeitpunkt der Inspiration und Exspiration selbst bestimmen. Die Atemhübe des Gerätes werden also mit den Ein- und Ausatembemühungen des Patienten synchronisiert. Der Atemhub des Gerätes erfolgt volumen- oder druckgeregelt. Das Beatmungsgerät schaltet bei der assistierten, druckgeregelten Beatmung synchron zu den Atembemühungen des Patienten zwischen einem voreingestellten inspiratorischen und einem expiratorischen Druckniveau.

Die assistierte Beatmung im sogenannten S-Modus erlaubt die freie Umschaltung zwischen inspiratorischem und exspiratorischem Druck (IPAP, EPAP) abhängig von durch den Patienten initiierten Atemhüben (Trigger durch die Spontanatmung)

Bei der assistierten Beatmung im sogenannten ST-Modus beschreibt der ST-Modus eine Mischform aus Unterstützung der spontanen Atmung (S- Modus) und mandatorischer Beatmung. Es wird eine Hintergrundfrequenz festgelegt, über die der Mindestabstand zwischen Atemintervallen definiert ist. Der Patient hat die Möglichkeit, innerhalb dieser Intervalle (aus einer Exspirationsphase) eine Inspiration, d.h. Umschaltung in den IPAP durch Atemanstrengung auszulösen. Erfolgt bis zur Erreichung der durch die Hintergrundfrequenz definierten maximalen erlaubten Intervall-Länge keine Triggerung durch den Patienten, löst das Beatmungsgerät die Umschaltung auf das inspiratorische Druckniveau aus, um so einen Atemhub des Patienten zu provozieren. Der Modus ermöglicht ebenfalls eine mandatorische Beamtung, während der der Patient zusätzliche Atemhübe abfordern kann.

In einem weiteren Beatmungsverfahren triggert der Patient durch seine Atemanstrengung die Umschaltung auf den inspiratorischen Druck. Es verbleibt ihm jedoch keine Freiheit in Bezug auf die Exspiration. Nach einer festgelegten Inspirationszeit erfolgt zwangsläufig die Umschaltung auf das exspiratorische Druckniveau.

Bekannt ist auch bereits die Verwendung eines festen Zeitfensters (Tmin/Tmax). Es wird ein fixes Zeitfenster unter Angabe einer minimalen und einer maximalen Inspirationszeit vorgegeben, innerhalb derer der Patient durch Ausatemanstrengung eine Umschaltung auf das exspiratorische Druckniveau auslösen kann. Diese minimale und maximale Inspirationszeit werden am Gerät eingestellt und sind während der Therapie für den Patienten fix.
Eine Vorrichtung zur Beatmung nach dem Oberbegriff des Anspruchs 1 ist beispielsweise aus dem Dokument EP-A-0943 353 bekannt.

Aufgabe der vorliegenden Erfindung ist es, eine Vorrichtung der einleitend genannten Art derart- zu konstruieren, daß für den Patienten ein gesteigerter Benutzungskomfort bereitgestellt wird.

Diese Aufgabe wird durch eine Vorrichtung mit den Merkmalen des Anspruchs 1 gelöst.

Vorteilhafte Ausführungsformen der Erfindung sind Gegenstand der Unteransprüche. Durch die Ansteuerung der Atemgasquelle in Abhängigkeit von einem vorgegebenen adaptiv veränderbaren Akzeptanzfenster wird die Gefahr einer Asynchronisation zwischen dem Beatmungsgerät und der Patientenatmung vermindert. Es wird darüber hinaus eine verbesserte Beatmung durch ein Führen auf die Zielgröße erreicht. Das Akzeptanzfenster kann für eine Steuerung des Inspirationsbeginns sowie die Inspirationsendes als ein zeitfenster realisiert sein. Weicht die spontane Inspirationsdauer über eine längere Zeit von der Zielgröße ab, so wird die entsprechende zugelassene Abweichung vom Vorgabewert automatisch durch die Adaptionseinrichtung schrittweise in Richtung des Vorgabewertes verändert, so daß die Inspirationszeit in Richtung der Zielgröße verändert wird.

Durch die adaptive Veränderung mindestens eines Grenzwertes des Akzeptanzfensters vorzugsweise in Abhängigkeit von einer vorgenommenen Mittelwertbildung wird zum einen gewährleistet, daß die Funktion des Beatmungsgerätes innerhalb eines vorgegebenen Bereiches liegt. Innerhalb dieses zulässigen Bereiches erfolgt eine weitgehende Adaption an die natürliche Atemtätigkeit des Patienten. Durch die adaptive Veränderung der Grenzen des Akzeptanzfensters werden dem Patienten bei einer über einen längeren Zeitraum als mit den Vorgaben übereinstimmend klassifizierten Atemtätigkeiten für einzelne Atemzüge größere Abweichungen von den Vorgabewerten erlaubt. Liegen beispielsweise eine größere Anzahl ausreichend langer Atemzüge vor, so wird eine kurze Spontanatmung nicht vom Gerät übersteuert, da im Mittel eine akzeptable Atmung vorliegt.

Bei einem zeitlichen Atemparameter entspricht die Bezeichnung, daß der Atemparameter unterhalb eines Grenzwertes bzw. Toleranzwertes liegt, dem sprachüblichen "vor" oder "früher als" ein vorgegebener Zeitpunkt, die Bezeichnung als oberhalb eines Grenzwertes liegend entspricht dem sprachüblichen "nach" oder "später als" ein vorgegebener Zeitpunkt.

Eine universelle Mittelwertbildung wird dadurch bereitgestellt, daß ein Mittelwertbildner für eine lineare Mittelwertbildung ausgebildet ist.

Eine weitere Verbesserung des Steuerungsverhaltens kann dadurch erreicht werden, daß der Grenzwertspeicher Grenzwerte für eine Atemfrequenz bevorratet.

Ebenfalls können Grenzwerte für das Verhältnis der Zeitdauern der Inspirationsphasen und der Exspirationsphasen bevorratet werden.

Darüber hinaus trägt es zu einer verbesserten Steuerung bei, daß der Grenzwertspeicher Grenzwerte für den Startzeitpunkt der Inspiration beinhaltet.

Ebenfalls ist daran gedacht, daß der Grenzwertspeicher Grenzwerte für den Endzeitpunkt der Inspiration beinhaltet.

Eine nochmals gesteigerte Benutzungsfreundlichkeit wird dadurch erreicht, daß die Adaptionseinrichtung zur Veränderung mindestens eines Grenzwertes für den Inspirationsbeginn ausgebildet ist. Nachfolgend werden die Begriffe Inspirationsbeginn und Exspirationsende sowie Exspirationsende und Inspirationsbeginn jeweils als synonyme Bezeichnungen verwendet.

Ebenfalls ist daran gedacht, daß die Adaptionseinrichtung zur Veränderung mindestens eines Grenzwertes für das Inspirationsende ausgebildet ist.

Eine zusätzliche Adaptionsmöglichkeit wird dadurch bereitgestellt, daß die Adaptionseinrichtung zur Veränderung mindestens eines Grenzwertes für das Atemfrequenzfenster ausgebildet ist.

Eine weitere Verbesserung des Systemverhaltens kann dadurch erreicht werden, daß die Adaptionseinrichtung zur Berücksichtigung einer zielgröße für das Tidalvolumen ausgebildet ist.

In den Zeichnungen sind Ausführungsbeispiele der Erfindung schematisch dargestellt. Es zeigen:
- Fig. 1: eine perspektivische Darstellung eines Beatmungsgerätes mit Verbindungsschlauch zu einer Beatmungsmaske,
- Fig. 2: eine schematische Darstellung der wesentlichen funktionellen Komponenten,
- Fig. 3: ein Druck-Zeit-Diagramm zur veranschaulichung des zeitlichen Verlaufes der Spontanatmung mit einem Exspirationsbeginn des Patienten innerhalb eines von der Steuerung vorgegebenen Toleranzfensters sowie einem aktuellen adaptiven Zeitfenster, innerhalb dessen ein individueller Exspirationsbeginn des Patienten von der Beatmungssteuerung nicht korrigiert wird,
- Fig. 4: eine Darstellung entsprechend Fig. 3 für einen Beginn der Inspiration mit zugeordnetem Toleranzfenster sowie adaptivem Zeitfenster,
- Fig. 5: ein Diagramm zur veranschaulichung einer Verkleinerung eines adaptiven Zeitfensters bei einem verfrühten Exspirationsbeginn durch den Patienten außerhalb des Toleranzfensters,
- Fig. 6: ein gegenüber Fig. 5 nochmals verkleinertes Zeitfenster bei wiederholter verfrühter Exspiration außerhalb des Toleranzfensters,
- Fig. 7: ein gegenüber der Darstellung in Fig. 4 verkleinertes Zeitfenster bei verspäteter Exspiration außerhalb des Toleranzfensters,
- Fig. 8: ein gegenüber Fig. 7 nochmals verkleinertes Zeitfenster bei wiederholter verspäteter Exspiration,
- Fig. 9: ein gegenüber der Darstellung in Fig. 4 verkleinertes Zeitfenster bei verspäteter Inspiration außerhalb des Toleranzfensters,
- Fig. 10: ein gegenüber Fig. 9 nochmals verkleinertes Zeitfenster bei wiederholter verspäteter Inspiration,
- Fig. 11: ein Ablaufdiagramm zur veranschaulichung des grundsätzlichen Steuerungsablaufes,
- Fig. 12: ein Ablaufdiagramm zur Veranschaulichung der steuerungstechnischen Berechnung der jeweiligen Zeitfenstergröße und
- Fig. 13: ein Ablaufdiagramm zur veranschaulichung der steuerungstechnischen Abläufe bei einem Wechsel der Atemphase.

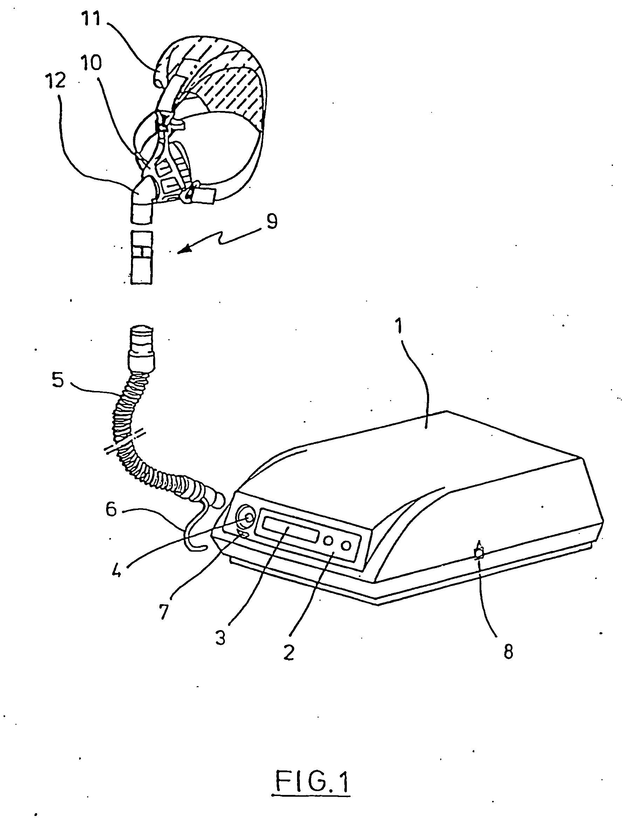

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus eine Beatmungsmaske (10), die als Nasalmaske ausgebildet ist. Gemäß einer anderen Ausführungsform kann auch eine Vollgesichtsmaske verwendet werden. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

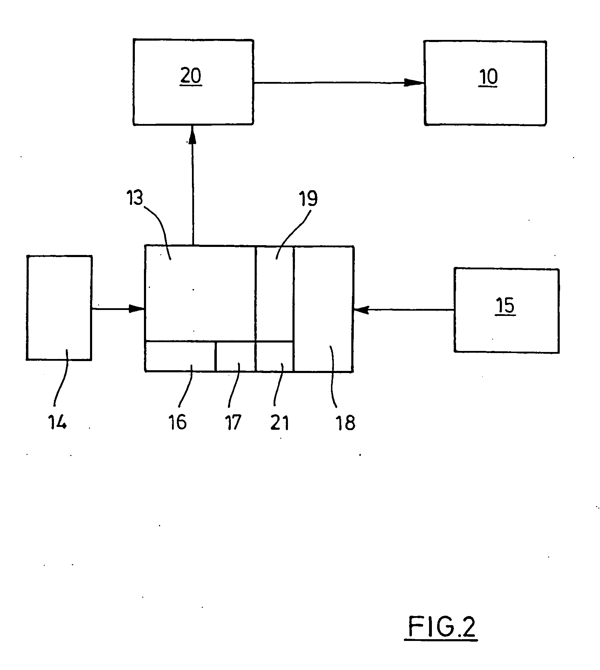

Fig. 2 zeigt den prinzipiellen Aufbau der steuerungstechnischen vorrichtungskomponenten. Eine Steuereinheit (13) ist mit einem Eingabemodul (14) zur Dateneingabe versehen. Über das Eingabemodul (14) können beispielsweise von einem Arzt Sollwerte für die Beatmung eingegeben werden. Die Steuereinheit (13) ist mit einem Sensor (15) verbunden, der mindestens einen Beatmungsparameter eines Patienten erfaßt.

Die Steuereinheit (13) ist darüber hinaus mit einem als Zeitfensterspeicher (16) ausgebildeten Grenzwertspeicher für Vorgabewerte eines aktuell zulässigen frühesten sowie spätesten Beginns der Inspirationsphase sowie mit einem Toleranzfensterspeicher (17) versehen, der maximal zulässige untere und obere Abweichungen von einem Idealzeitpunkt des Inspirationsbeginns enthält. Die Meßdaten des Sensors (15) werden einem Mittelwertbildner (18) zugeführt, der mindestens einen Kennwert der tatsächlichen Inspirationszeiten über eine vorgebbare Zeitspanne mittelt. Insbesondere ist daran gedacht, die Mittelwertbildung sowohl hinsichtlich des zeitpunktes des tatsächlichen Inspirationsbeginns, der Zeitdauer der Inspiration sowie des Endzeitpunktes der Inspiration durchzuführen. Ebenfalls kann sich die Mittelwertbildung auf die Beatmungsfrequenz bzw. das Verhältnis aus Inspirationsdauer zu Exspirationsdauer (I:E-Verhältnis) beziehen.

Ergänzend zum Toleranzfensterspeicher (17) für den Inspirationsbeginn kann auch ein Toleranzfensterspeicher (21) für das Exspirationsende vorgesehen werden. Durch die Überwachung des Inspirationsbeginns sowie des Inspirationsendes werden auch die entsprechenden komplementären Werte für die Exspiration mit erfaßt, da das Inspirationsende gleich dem Exspirationsbeginn und der Inspirationsbeginn gleich dem Exspirationsende ist.

In Abhängigkeit von den Auswertungsergebnissen des Mittelwertbildners (18) nimmt eine Adaptionseinrichtung (19) eine Modifikation der Werte im Zeitfensterspeicher (16) vor. In Abhängigkeit von den jeweils vorliegenden Meßwerten sowie den Grenzwerten aus dem Toleranzfensterspeicher (17, 21) steuert die Steuereinheit (13) eine Atemgasquelle (20), die mit der Beatmungsmaske (10) verbunden ist. Die Steuerung erfolgt dabei derart, daß bei Vorliegen der Auslösevoraussetzungen von der Atemgasquelle (20) ein inspiratorisches Druckniveau generiert wird.

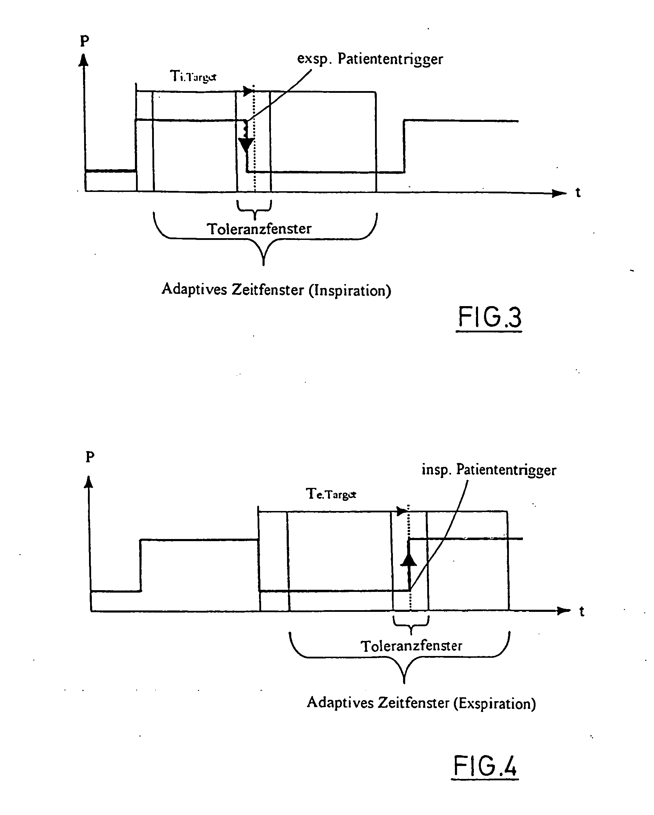

Fig. 3 zeigt schematisch in einem Druck-Zeit-Diagramm den Druckverlauf zwischen dem Inspirationsdruck sowie dem Exspirationsdruck. Es ist zu erkennen, daß von der Gerätesteuerung lediglich zwei unterschiedliche Druckniveaus vorgegeben werden. Eine Umschaltung zwischen dem inspiratorischem Druck und dem expiratorischen Druck erfolgt innerhalb der definierten Zeitfenster in Abhängigkeit von einer sensorisch detektierten expiratorischen Anstrengung des Patienten. Liegt der Beginn der expiratorischen Anstrengung innerhalb des vorgegebenen Zeitfensters, so wird der Druckwechsel hierdurch getriggert.

Fig. 3 zeigt insbesondere das adaptive Zeitfenster für das Ende der Inspiration. In der Mitte des Toleranzfensters ist punktiert der ideale Zeitpunkt für das Ende der Inspiration und somit den Exspirationsbeginn eingezeichnet. Abweichungen der tatsächlichen Atemanstrengung des Patienten innerhalb des Toleranzfensters von dem Idealwert führen nicht zu einer adaptiven Veränderung des adaptiven Zeitfensters, wenn dieses bereits seine maximale Größe aufweist. Besitzt das Zeitfenster aufgrund gegebenenfalls im Vorverlauf aufgetretener Abweichungen der spontanen Patientenatmung vom vorgegebenen Idealverlauf eine verringerte Größe, so führt eine Spontanatmung innerhalb des Toleranzfensters zu einer schrittweisen vergrößerung des adaptiven Zeitfensters.

Bei einer expiratorischen Atemanstrengung des Patienten innerhalb des adaptiven Zeitfensters wird zum Zeitpunkt der expiratorischen Atemanstrengung des Patienten von der Gerätesteuerung die Exspiration ausgelöst, ohne daß eine steuerungstechnische Abweichung vom durch den Patienten vorgegebenen Atmungszeitpunkt vorgenommen wird.

Fig. 4 zeigt entsprechend der Darstellung in Fig. 3 ein adaptives Zeitfenster für den Beginn der Inspiration und somit für das Ende der Exspiration. Ebenfalls ist ein Toleranzfenster für die inspiratorische Spontanatmung des Patienten dargestellt. Auch hinsichtlich des Beginns der Inspiration werden alle vom Patienten vorgegebenen Zeitpunkte innerhalb des adaptiven Zeitfensters von der Gerätesteuerung unmittelbar in einen Beginn der Inspiration umgesetzt.

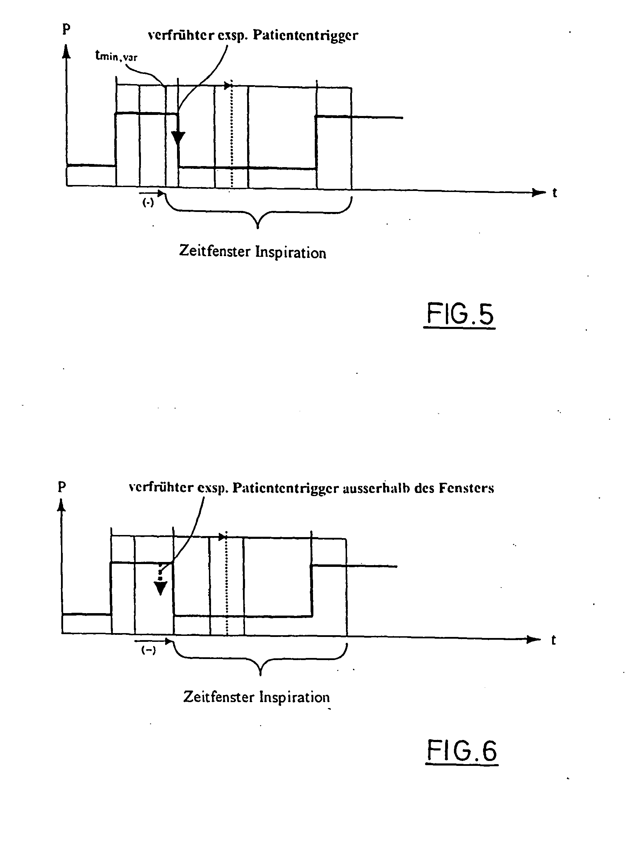

Fig. 5 zeigt eine verkleinerung des adaptiven Zeitfensters für den Beginn der Exspiration bei einer verfrühten expiratorischen Spontanatmung des Patienten außerhalb des Toleranzfensters. Eine entsprechende verfrühte Exspiration des Patienten führt dazu, daß das adaptive Zeitfenster an seinem linken Rand, d.h. hinsichtlich des frühesten zugelassenen Exspirationszeitpunktes, verkleinert wird. Bei der wiederholten verfrühten Exspiration wird somit die Toleranz der Gerätesteuerung hinsichtlich weiterer verfrühter Exspirationen vermindert. Bei der in Fig. 5 dargestellten Beatmungssituation liegt aber die spontane Patientenexpiration auch noch innerhalb des verkleinerten adaptiven Zeitfensters, so daß die Gerätesteuerung noch zu dem vom Patienten vorgegebenen Zeitpunkt die Exspiration einleitet.

Fig. 6 zeigt die Lage des adaptiven zeitfensters nach weiteren verfrühten Exspirationen des Patienten. Das adaptive Zeitfenster wurde nunmehr hinsichtlich der zugelassenen frühesten Exspiration noch mehr verkleinert, so daß der von der Spontanatmung des Patienten vorgegebene Exspirationsbeginn außerhalb des adaptiven zeitfensters liegt. Dies führt dazu, daß die Gerätesteuerung diese externe Triggerung durch den Patienten nicht mehr akzeptiert, sondern die Exspiration erst zu dem vom adaptiven Zeitfenster zugelassenen frühesten auf den vom Patienten gewünschten Zeitpunkt folgenden Zeitpunkt durchführt.

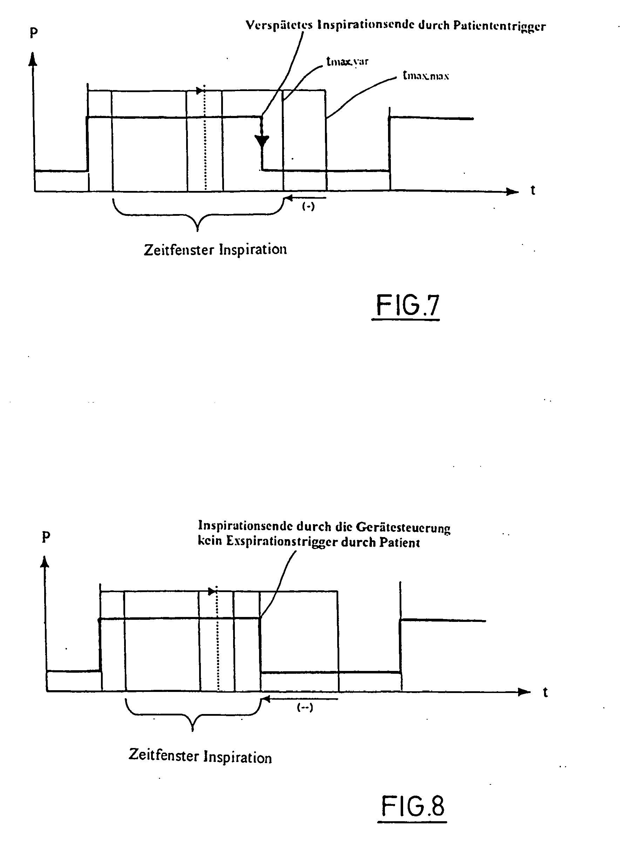

Fig. 7 zeigt eine gegenüber Fig. 3 veränderte Lage des adaptiven Zeitfensters nach einem wiederholten verspäteten Inspirationsende außerhalb des Toleranzfensters. Aufgrund der verspäteten Inspirationsenden wurde hier das Toleranzfenster am rechten Rand, dies bedeutet hinsichtlich des spätesten von der Gerätesteuerung noch akzeptierten Inspirationsendes, verkürzt. Die vom Patienten vorgegebene externe Triggerung für das Inspirationsende liegt jedoch noch innerhalb des aktuellen adaptiven Zeitfensters und wird deshalb von der Gerätesteuerung noch akzeptiert.

Gemäß der Darstellung in Fig. 8 wurde gegenüber Fig. 7 das adaptive Zeitfenster hinsichtlich des spätesten zugelassenen Inspirationsendes nochmals verkürzt. Innerhalb des dargestellten Zeitfensters konnte kein externer Exspirationstrigger des Patienten sensorisch erkannt werden, so daß von der Gerätesteuerung zum Ende des Zeitfensters die Inspiration beendet und die Exspiration eingeleitet wird.

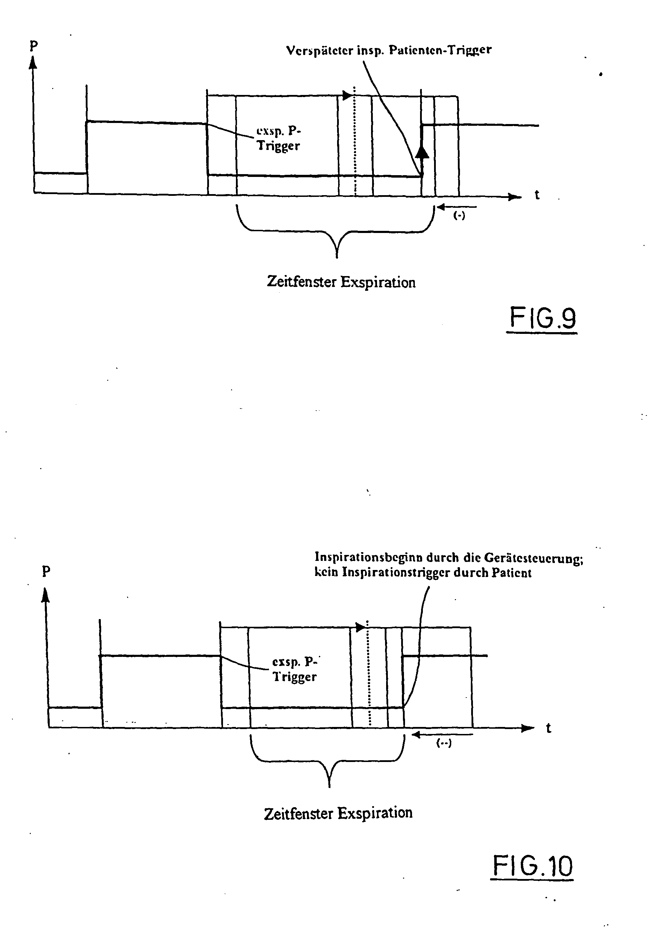

Fig. 9 zeigt hinsichtlich des Beginns der Inspiration eine gegenüber Fig. 4 abgewandelte Lage des zeitfensters. Als Folge einer verspäteten Inspiration durch den Patienten wurde das Zeitfenster im rechten Bereich, d.h. hinsichtlich des spätesten noch zugelassenen Inspirationszeitpunktes, verkürzt. Die am Patienten detektierte inspiratorische Atemanstrengung liegt jedoch noch innerhalb des aktuellen Zeitfensters, so daß die Exspirationstriggerung durch den Patienten von der Gerätesteuerung akzeptiert und die Inspirationsanstrengung durch den Patienten von der Gerätesteuerung akzeptiert und somit die Inspiration zum vom Patienten gewünschten Zeitpunkt eingeleitet wird.

Fig. 10 zeigt eine gegenüber Fig. 9 nochmals verkleinerte Dimensionierung des Zeitfensters aufgrund wiederholter verspäteter Inspirationen. Innerhalb des dargestellten Zeitfensters konnte von der Gerätesteuerung kein inspiratorischer Trigger des Patienten detektiert werden, so daß zum Endzeitpunkt des adaptiven Zeitfensters die Inspiration von der Gerätesteuerung eingeleitet wird.

Das I:E-Verhältnis der Spontanatmung beeinflußt den Steuervorgang. Eine Adaptionseinrichtung, beispielsweise ein Regler, verkleinert das Zeitfenster für die Umschaltung von der Inspiration in die Exspiration, wenn der Patient über längere Zeit weit entfernt von der Zielgröße atmet, so daß das Gerät den Patienten mit seiner Atmung in diesem Fall zur Zielgröße "zieht". Alternativ oder ergänzend zu einer Verkleinerung kann auch eine Verschiebung des Zeitfensters erfolgen. Atmet der Patient in der Nähe des festgelegten T_{insp-target}, wird das Zeitfenster wieder vergrößert, so daß für einzelne Atemzüge eine große Flexibilität zur Verfügung steht. So sind beispielsweise lange Seufzeratemzüge möglich oder einzelne kurze Atemzüge, wenn die Atmung sonst in der Nähe der Zielgröße liegt. Auch im Zusammenhang mit der Vergrößerung des Zeit fensters kann alternativ oder ergänzend eine Verschiebung erfolgen.

Weiterhin ist es möglich, eine Zielgröße für das Tidalvolumen zu definieren. Weicht das aktuelle Tidalvolumen über eine gewisse Zeit nach unten vom Zielvolumen ab und liegt gleichzeitig die Inspirationszeit unterhalb der Zielgröße, wird ebenfalls das Fenster für die Inspirationsdauer verkleinert und der Patient wird zur Zielgröße gezogen.

Gemäß einer weiteren Ausführungsform ist es möglich, daß bei Erreichen der Zielgröße für das Tidalvolumen und verkürzter Inspirationszeit das Zeitfenster nicht verkleinert wird, da eine ausreichende Ventilation durch das Tidalvolumen erreicht ist.

Weiterhin ist es denkbar, daß durch ein Tidalvolumen, das in der Nähe des Zielvolumens oder darüber liegt, das Zeitfenster für die Inspiration wieder vergrößert wird, der Patient also wieder eine größere Flexibilität bei der Umschaltung auf den exspiratorischen Druck erhält.

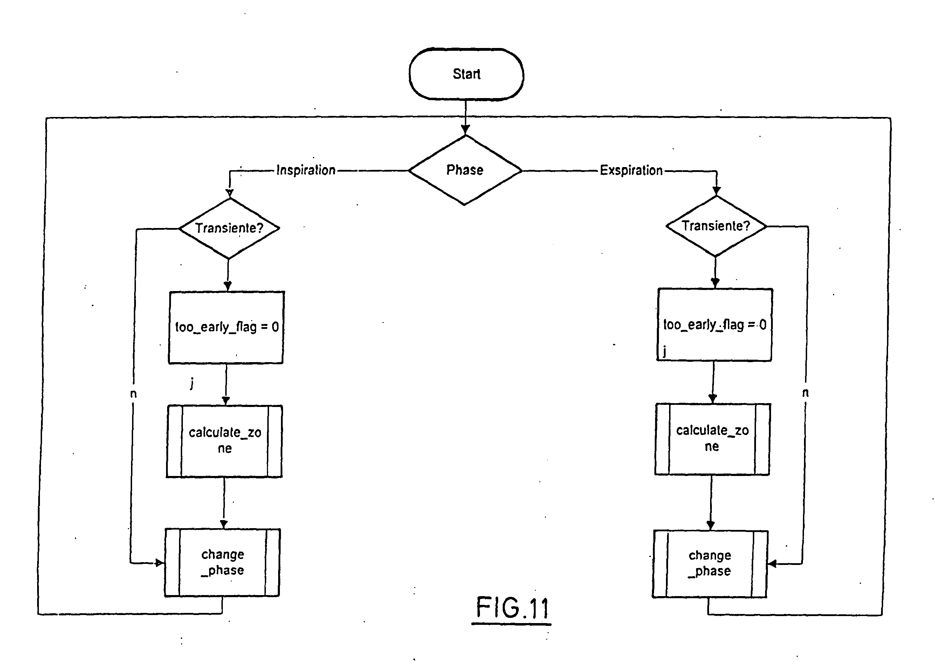

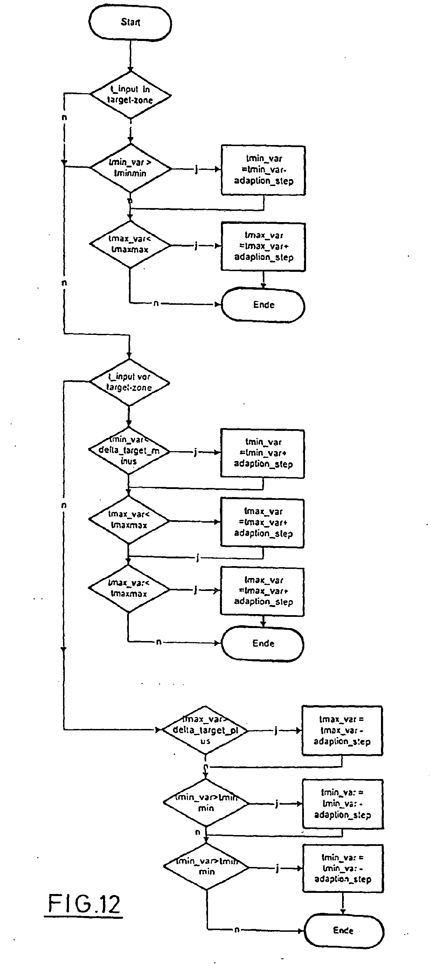

Fig. 11 zeigt ein Übersichtsschaubild zur Veranschaulichung der steuerungstechnischen Abläufe. Alternierend wird jeweils überprüft, ob eine Inspirationsphase oder eine Exspirationsphase vorliegt. Bei jeder der beiden Erkennungsfälle wird zunächst überprüft, ob ein Phasenübergang vorliegt. Ist dies der Fall, wird zunächst eine Kennung, daß möglicherweise eine zu frühe Auslösung vorliegt, zurückgesetzt, anschließend werden ein Kalkulationsmodul und ein Phasenänderungsmodul durchlaufen. Der steuerungstechnische Ablauf für diese beiden Module wird in Fig. 12 näher erläutert. Zu Beginn einer neuen Atemphase werden mit Hilfe der letzten entsprechenden Phasendauer neue Grenzen festgelegt.

Fig. 12 veranschaulicht einen möglichen Steuerungsablauf für das Kalkulationsmodul. Grundsätzlich werden die Grenzen des adaptiven Zeitintervalles aufgeweitet, wenn die Atmungsparameter innerhalb des Toleranzintervalles liegen, bis die maximale Aufweitung des adaptiven Zeitintervalls erreicht ist. Ergibt die Signalauswertung, daß das Ende der letzten Inspiration zu früh erfolgte, so wird die untere Grenze des adaptiven Zeitintervalles angepaßt. Eine Erweiterung der oberen Grenze des adaptiven Zeitintervalles erfolgt relativ schnell, um dem Patienten längere Inspirationen zu ermöglichen. Auch in umgekehrter Richtung erfolgt eine kurzfristige adaptive Anpassung.

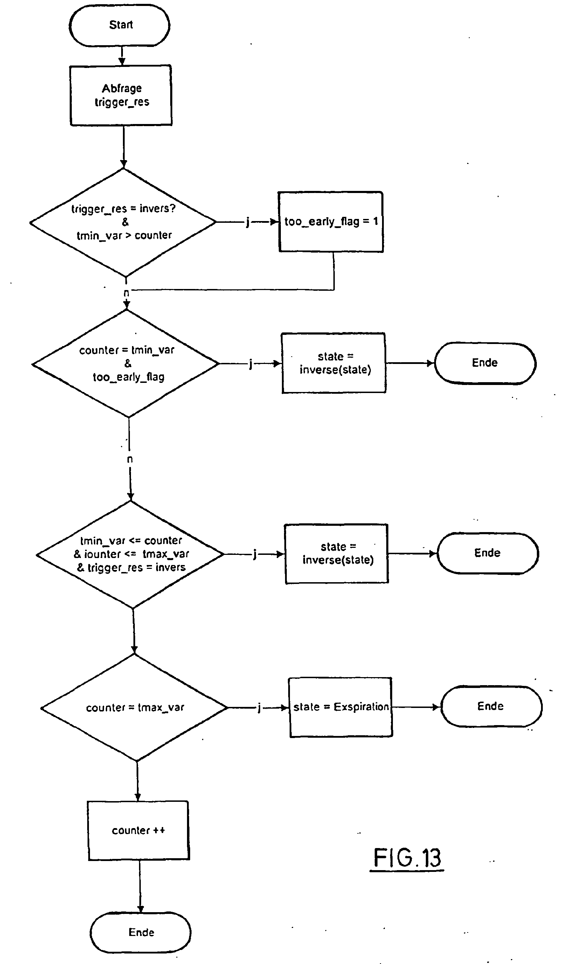

Bei dem in Fig. 13 veranschaulichten Phasenwechselmodul wird zunächst abgefragt, ob ein inverser Triggerimpuls detektiert wurde. Der Detailablauf ergibt sich aus den im Ablaufdiagramm vorgesehenen Beschriftungen.

## Patentansprüche

1. Vorrichtung zur Beatmung, die eine Atemgasquelle (20), eine Steuereinrichtung (13) sowie eine Anschlußeinrichtung zur Verbindung mit einer Beatmungsmaske aufweist und bei der die Steuereinrichtung an mindestens einen Sensor zur Erfassung eines Meßparameters angeschlossen ist, wobei der Sensor (15) zu Erfassung eines Atemparameters des Patienten ausgebildet ist und die Steuereinrichtung (13) einen Grenzwertspeicher (16) für Grenzwerte eines Akzeptanzfensters für einen Beatmungsparameter aufweist, wobei die Steuereinrichtung (13) sowohl einen die Atemgasquelle (20) bei einer Detektion des Atemparameters innerhalb des Akzeptanzfensters in Abhängigkeit vom Sensorsignal ansteuernden Triggermodus als auch einen für einen außerhalb des Akzeptanzfensters liegenden Atemparameter aktivierten mandatorischen Steuermodus aufweist, **dadurch gekennzeichnet, daß** die Steuereinrichtung (13) eine Adaptionseinrichtung (19) zur Veränderung der Grenzwerte in Abhängigkeit vom Meßsignal des Sensors (15) aufweist und daß die Steuereinrichtung (13) einen Inkrementor zur Erhöhung eines unteren Grenzwertes bei ei-nem unterhalb eines ersten Toleranzwertes detektierten Atemparameter sowie einem Dekrementor zur Verminderung eines oberen Grenzwertes bei einem oberhalb eines zweiten Toleranzwertes detektierten Atemparameter aufweist.

2. Vorrichtung nach Anspruch 1, **dadurch gekennzeichnet, daß** die Steuereinrichtung (13) einen Mittelwertbildner (18) aufweist.

3. Vorrichtung nach Anspruch 2, **dadurch gekennzeichnet, daß** der Mittelwertbildner (18) für eine lineare Mittelwertbildung ausgebildet ist.

4. Vorrichtung nach einem der Ansprüche 1 bis 3, **dadurch gekennzeichnet, daß** der Grenzwertspeicher (16) Grenzwerte für den Startzeitpunkt der Inspiration bevorratet.

5. Vorrichtung nach einem der Ansprüche 1 bis 3, **dadurch gekennzeichnet, daß** der Grenzwertspeicher (16) Grenzwerte für den Endzeitpunkt der Inspiration bevorratet.

6. Vorrichtung nach einem der Ansprüche 1 bis 5, **dadurch gekennzeichnet, daß** der Grenzwertspeicher (16) Grenzwerte für eine Atemfrequenz bevorratet.

7. Vorrichtung nach einem der Ansprüche 1 bis 5, **dadurch gekennzeichnet, daß** der Grenzwertspeicher (16) Grenzwerte für ein I:E-Verhältnis bevorratet.

8. Vorrichtung nach einem der Ansprüche 1 bis 7, **dadurch gekennzeichnet, daß** die Adaptionseinrichtung (19) zur Veränderung mindestens eines Grenzwertes für den Inspirationsbeginn ausgebildet ist.

9. Vorrichtung nach einem der Ansprüche 1 bis 7, **dadurch gekennzeichnet, daß** die Adaptionseinrichtung (19) zur Veränderung mindestens eines Grenzwertes für das Inspirationsende ausgebildet ist.

10. Vorrichtung nach einem der Ansprüche 1 bis 7, **dadurch gekennzeichnet, daß** die Adaptionseinrichtung (19) zur Veränderung mindestens eines Grenzwertes für die Inspirationsdauer ausgebildet ist.

11. Vorrichtung nach einem der Ansprüche 1 bis 7, **dadurch gekennzeichnet, daß** die Adaptionseinrichtung (19) zur Veränderung mindestens eines Grenzwertes für die Atemfrequenz ausgebildet ist.

12. Vorrichtung nach einem der Ansprüche 1 bis 7, **dadurch gekennzeichnet, daß** die Adaptionseinrichtung (19) zur Veränderung mindestens eines Grenzwertes für das I:E-Verhältnis ausgebildet ist

13. Vorrichtung nach einem der Ansprüche 1 bis 7, **dadurch gekennzeichnet, daß** die Adaptionseinrichtung (19) zur Berücksichtigung einer Zielgröße für das Tidalvolumen ausgebildet ist.

14. Vorrichtung nach einem der Ansprüche 1 bis 7, **dadurch gekennzeichnet, daß** die Adaptionseinrichtung (19) zur Veränderung mindestens eines Grenzwertes für den Exspirationsbeginn ausgebildet ist.

15. Vorrichtung nach einem der Ansprüche 1 bis 7, **dadurch gekennzeichnet, daß** die Adaptionseinrichtung (19) zur Veränderung mindestens eines Grenzwertes für das Exspirationsende ausgebildet ist.

16. Vorrichtung nach einem der Ansprüche 1 bis 7, **dadurch gekennzeichnet, daß** die Adaptionseinrichtung (19) zur Veränderung mindestens eines Grenzwertes für die Exspirationsdauer ausgebildet ist.

## Claims

1. Breathing device, comprising a respiratory gas source (20), a controller (13) and a connecting device for connection to a respiratory mask, and in which the controller is connected to at least one sensor for detecting a measuring parameter, wherein the sensor (15) is constructed to detect a respiratory parameter of the patient and the controller (13) comprises a limiting value memory (16) for limiting values of an acceptance window for a respiratory parameter, wherein the controller (13) comprises a trigger mode that activates the respiratory gas source (20) on detection of the respiratory parameter within the acceptance window and as a function of the sensor signal, and a mandatory control mode activated for a respiratory parameter that lies outside of the acceptance window, **characterised in that** the controller (13) comprises an adapter (19) for changing the limiting values as a function of the measuring signal of the sensor (15), and **in that** the controller (13) comprises a incrementor for increasing a lower limiting value in the case of a respiratory parameter detected below a first tolerance value, and a decrementor for reducing an upper limiting value in the case of a respiratory parameter detected above a second tolerance value.

2. Device according to claim 1, **characterised in that** the controller (13) comprises a mean value generator (18).

3. Device according to claim 2, **characterised in that** the mean value generator (18) is constructed for linear mean value generation.

4. Device according to any one of claims 1 to 3, **characterised in that** the limiting value memory (16) stores limiting values for the start time of inspiration.

5. Device according to any one of claims 1 to 3, **characterised in that** the limiting value memory (16) stores limiting values for the end time of inspiration.

6. Device according to any one of claims 1 to 5, **characterised in that** the limiting value memory (16) stores limiting values for a breathing rate.

7. Device according to any one of claims 1 to 5, **characterised in that** the limiting value memory (16) stores limiting values for an I:E ratio.

8. Device according to any one of claims 1 to 7, **characterised in that** the adapter (19) is constructed to change at least one limiting value for the start of inspiration.

9. Device according to any one of claims 1 to 7, **characterised in that** the adapter (19) is constructed to change at least one limiting value for the end of inspiration.

10. Device according to any one of claims 1 to 7, **characterised in that** the adapter (19) is constructed to change at least one limiting value for the period of inspiration.

11. Device according to any one of claims 1 to 7, **characterised in that** the adapter (19) is constructed to change at least one limiting value for the breathing rate.

12. Device according to any one of claims 1 to 7, **characterised in that** the adapter (19) is constructed to change at least one limiting value for the I:E ratio.

13. Device according to any one of claims 1 to 7, **characterised in that** the adapter (19) is constructed to take account of a target value for the tidal volume.

14. Device according to any one of claims 1 to 7, **characterised in that** the adapter (19) is constructed to change at least one limiting value for the start of expiration.

15. Device according to any one of claims 1 to 7, **characterised in that** the adapter (19) is constructed to change at least one limiting value for the end of expiration.

16. Device according to any one of claims 1 to 7, **characterised in that** the adapter (19) is constructed to change at least one limiting value for the period of expiration.

## Revendications

1. Dispositif respiratoire qui présente une source de gaz respiratoire (20), un système de commande (13) ainsi qu'un dispositif de raccordement pour le raccordement à un masque respiratoire, et dans lequel l'unité de commande est raccordée à au moins un détecteur pour la détection d'un paramètre de mesure, le détecteur (15) étant conçu pour la détection d'un paramètre de respiration du patient, et le système de commande (13) présentant une mémoire de valeur limite (16) pour des valeurs limites d'une fenêtre d'acceptation pour un paramètre de respiration, le système de commande (13) présentant aussi bien un mode de déclenchement excitant la source de gaz respiratoire (20) lors d'une détection du paramètre de respiration à l'intérieur de la fenêtre d'acceptation en fonction du signal de détecteur, qu'un mode de commande impératif, activé pour un paramètre situé en dehors de la fenêtre d'acception,
**caractérisé en ce que**
le système de commande (13) présente un système d'adaptation (19) pour la modification des valeurs limites en fonction du signal de mesure du détecteur (15), et que le système de commande (13) présente un organe d'incrémentation pour l'augmentation d'une valeur limite inférieure lors de la détection d'un paramètre de respiration situé au-dessous d'une première valeur de tolérance, ainsi qu'un organe de décrémentation pour la réduction d'une valeur limite supérieure lors de la détection d'un paramètre de respiration situé au-dessus d'une deuxième valeur de tolérance.

2. Dispositif respiratoire selon la revendication 1, **caractérisé en ce que** le système de commande (13) présente un organe établissant la moyenne (18).

3. Dispositif respiratoire selon la revendication 2, **caractérisé en ce que** l'organe établissant la moyenne (18) est conçu pour un établissement linéaire de la valeur moyenne.

4. Dispositif respiratoire selon l'une des revendications 1 à 3, **caractérisé en ce que** la mémoire de valeurs limites (16) mémorise des valeurs limites pour le moment auquel commence l'inspiration.

5. Dispositif respiratoire selon l'une des revendications 1 à 3, **caractérisé en ce que** la mémoire de valeurs limites (16) mémorise des valeurs limites pour le moment auquel se termine l'expiration.

6. Dispositif respiratoire selon l'une des revendications 1 à 5, **caractérisé en ce que** la mémoire de valeurs limites (16) mémorise des valeurs limites pour une fréquence de respiration.

7. Dispositif respiratoire selon l'une des revendications 1 à 5, **caractérisé en ce que** la mémoire de valeurs limites (16) mémorise des valeurs limites pour un rapport I:E.

8. Dispositif respiratoire selon l'une des revendications 1 à 7, **caractérisé en ce que** le dispositif d'adaptation (19) est conçu pour la modification d'au moins une valeur limite pour le début de l'inspiration.

9. Dispositif respiratoire selon l'une des revendications 1 à 7, **caractérisé en ce que** le dispositif d'adaptation (19) est conçu pour la modification d'au moins une valeur limite pour la fin de l'inspiration.

10. Dispositif respiratoire selon l'une des revendications 1 à 7, **caractérisé en ce que** le dispositif d'adaptation (19) est conçu pour la modification d'au moins une valeur limite pour la durée de l'inspiration.

11. Dispositif respiratoire selon l'une des revendications 1 à 7, **caractérisé en ce que** le dispositif d'adaptation (19) est conçu pour la modification d'au moins une valeur limite pour la fréquence de respiration.

12. Dispositif respiratoire selon l'une des revendications 1 à 7, **caractérisé en ce que** le dispositif d'adaptation (19) est conçu pour la modification d'au moins une valeur limite pour le rapport I:E.

13. Dispositif respiratoire selon l'une des revendications 1 à 7, **caractérisé en ce que** le dispositif d'adaptation (19) est conçu pour tenir compte d'une valeur cible pour le volume courant.

14. Dispositif respiratoire selon l'une des revendications 1 à 7, **caractérisé en ce que** le dispositif d'adaptation (19) est conçu pour la modification d'au moins une valeur limite pour le début de l'expiration.

15. Dispositif respiratoire selon l'une des revendications 1 à 7, **caractérisé en ce que** le dispositif d'adaptation (19) est conçu pour la modification d'au moins une valeur limite pour la fin de l'expiration.

16. Dispositif respiratoire selon l'une des revendications 1 à 7, **caractérisé en ce que** le dispositif d'adaptation (19) est conçu pour la modification d'au moins une valeur limite pour la durée de l'expiration.
